# EUROPEAN PATENT APPLICATION

(11) **EP 2 823 783 A1**
(43) Date of publication of application: **14.01.2015**
(21) Application number: 12870907.8
(22) Date of filing: 06.03.2012
(51) Int. Cl.: A61B 18/24

(54) **BIDIRECTIONAL SELF-CENTERING TIP FOR FIBER OPTICS DESIGNED FOR ENDOVASCULAR TREATMENTS**

(71) Applicant: INTERMEDIC ARFRAN, S.A., 08290 Cerdanyola (ES)
(72) Inventor: ARCUSA VILLACAMPA, Fco. Javier, E-08290 Cerdanyola del Vallès ( Barcelona) (ES)
(74) Representative: Morgades y Manonelles, Juan Antonio
(86) International application number: PCT/ES2012/070142
(87) International publication number: WO 2013/132112

(57) **Abstract**

The invention relates to a cable or optical fibre, to which a protective finish has been added, which is formed by a tubular duct, the end parts of which have been mechanized in the form of fingers that have elbowed areas in the vicinity of the tips thereof, which elbowed areas expand toward the outside when the cable and said covering protrude from the catheter, and contract when the cable and finish are introduced into the catheter, avoiding, as is the case at present, a situation in which the fingers are straight and flat, which can produce a tear in walls of the blood vessel in the event of the operator wishing to reinsert the optical fibre through a vein in order to resume treating an already treated area.

## Description

### Object of the Invention.

More specifically, the invention refers to an optical fibre that has a bidirectional self-centring tip, formed by a tubular duct, the ends of which have an improved configuration with regard to existing models, allowing the tip to move forward and backward inside a patient's blood vessel controlled by the operator, being much less harmful for the vessel.

### State of the Art.

On the market there are, and therefore can be considered as state of the art, different types of fibre optic cables especially designed for endovascular treatments, the purpose of which is the retraction or obliteration of the inner wall of blood vessels, avoiding at all times perforation of the vessels, which would have serious side effects such as paraesthesia, skin burns, and similar.

These fibre optic cables have different finishes on their end, to magnify and regulate the effects of the laser energy transmitted, and are adapted to cause the retraction or obliteration of the blood vessels they are inserted into, for which it should be possible, as it really is, to adjust the energy of the laser so it is correct in each case to the treatment being applied.

For instance, Patent PCT WO 2010/101464 describes and claims "A device for endovascular treatment and system for endovascular treatment and a method for operating the same", the main characteristic of which is that is has a guide coating, able to lead the fibre optic cable so that thanks to said coating, there is a safety distance between the cable, and the inner wall of the blood vessel, and it spreads out the energy emitted by the cable in a uniform circular crown shape, avoiding the end of the cable perforating the inner wall when the blood vessel curves.

However, the state of the art does not achieve a uniform forward and backward movement of the fibre optic cable for the operator to carry out endovascular treatment totally safely, when pulling back the cable, the operator forgets to carry out the corresponding shot or it has been insufficient to obliterate the vessel, it is very difficult to make the cable move forward again, inside the vessel without causing (sometime irreparable) damage to the blood vessels, with inevitable tears, while in turn this forward movement, if it occurs, deforms the jacket formed by sheets, some more than others, the safety distance between the cable and the inner wall of the blood vessel no longer being uniform.

### Scope of the Invention.

The invention presented here consists of a bidirectional self-centring tip for fibre optic cable, with the essential characteristic that it allows the movement of said tip forward and backward inside a blood vessel without causing any type of damage or lesion in the vessel, thus greatly facilitating the work of the operator.

### Description of the Invention.

This invention is characterised as defined in claim 1.

In one of the possible embodiments, a new tip is inserted in a conventional fibre optic cable, used for endovascular treatment with the help of an adjustable energy generator, which is transmitted through the fibre optic cable inside a blood vessel with the help of the corresponding catheter and an operator.

In this case, an optical fibre is preferably used that has the end where the laser energy is emitted on the inner wall of the blood vessel in a practically uniform conical shape.

The fibre optic cable has a coating that surrounds the protective jacket of the fibre optic cable. The coating is comprised of a tubular duct that may move forward and backwards inside a catheter, one of the ends of this coating having a bundle of tabs at the end of said tubular duct, that extend outward in a conical shape.

On the outside of these tabs there are respective end parts in the form of nails that are sloping with regard to the tabs towards the centre or longitudinal axis "e1" of the vessel.

Preferably the tabs in the end of the tubular duct have been machined in such a way that transversally they have concave side bases, and the ends of the tabs bend inwards forming elbowed end areas or nails, to facilitate forward and backward movement with regard to said tubular duct, avoiding any sort of interference between the points of these tabs and the inner wall of the blood vessel.

These angled areas are made in dimensions and with materials such that they expand outwards when projecting from the catheter, and contract when the optical fibre and its bidirectional self-centring tip are inserted into the catheter.

In addition, as this end part never interferes with the inner walls of the blood vessels, the optical fibre is really self-centred and 100% bidirectional.

Other details and characteristics shall be shown throughout the description below referring to drawings attached to this report which are shown for illustrative but not limiting purposes only in a drawing of the invention.

### Description of the drawings.

Figure 1 corresponds to a side elevation view of the outer wall (18) of a blood vessel (17) of a patient, and in whose inner wall (19) there is an optical fibre with its catheter (11), and around it the body of the bidirectional self-centring tip (13), the cable enveloping the jacket (12) of the optical fibre.
Figure 2 corresponds to:
   (a) a detailed side elevation view end part (20) of the body of the bidirectional self-centring tip (13), that ends in some tabs (16) that end in nails (15);
   (b) a detailed side elevation view of the body of the bidirectional self-centring tip (13), and its end part (20);
   (c) a bottom plan view of the tabs (16); and
   (d) a section through B-B' according to the view (c).

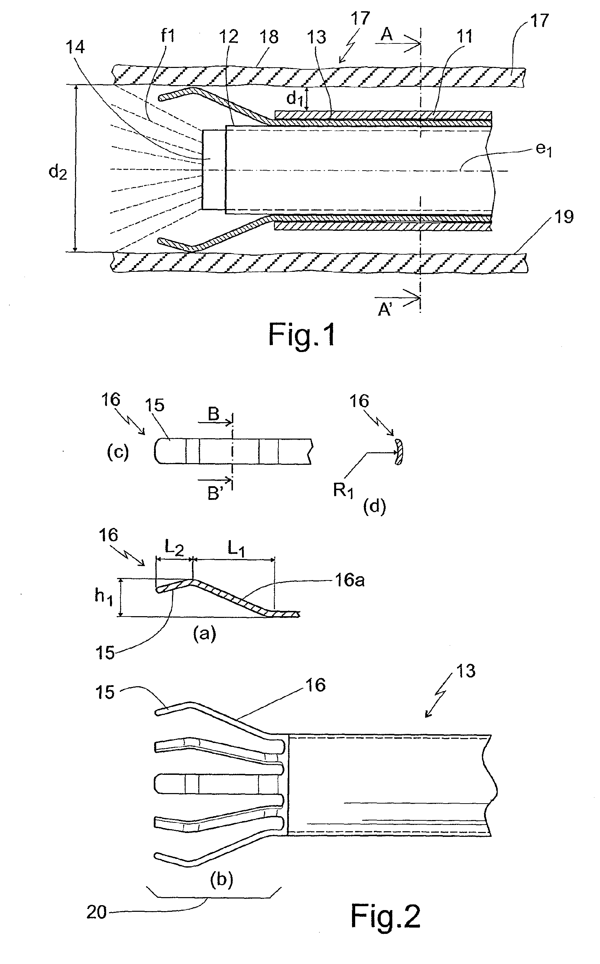

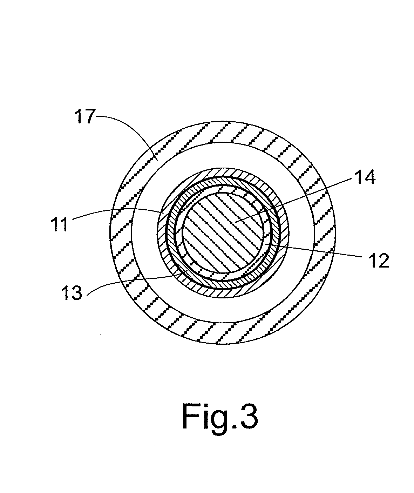

Figure 3 corresponds to a section through A-A' according to figure 1, in which from the outside to the inside you can see the blood vessel (17), the catheter (11), the body (13) of the coating, the jacket (12) and the fibre optic core (14) of the cable.

Below is a list of the different parts of the invention, which are indicated in the above drawings with their respective numbers; (11) catheter, (12) jacket, (13) body of the bidirectional self-centring tip, (14) core of the fibre optic cable, (15) nails, (16) tabs, (16a) inner portions of the tabs (16), (17) blood vessel, (18) outer wall, (19) inner wall, (20) end part.

### Description of an embodiment of the invention.

Generally, a fibre optic cable has a core and a cladding, which have different refraction indices. Both the core and the cladding are made of very rigid materials and as a result the fibre is extremely fragile. To make it flexible, it is plasticised with a jacket.

In one of the preferred embodiments of the invention, as can be seen in figure 1, the cable, as can be seen sectioned in figure 3, is formed by a core (14) with a cladding (12), the cladding (12) being surrounded by the flexible jacket (13), and the latter (13) sliding inside a catheter (11), and the catheter (11) slides inside a blood vessel (17) guided by the operator.

In the preferred embodiment, the optical fibre has an end where the laser energy is emitted on the inner wall of the blood vessel in a widening conical shape, see figure 1.

The blood vessel (17) or vein has an outer wall (18), and an inner wall (19), circulating inside the vessel (17), the catheter being uniformly separate at a distance d1 thanks to having a bidirectional self-centring tip (13) of tubular duct shape, the end part (20) or tip of which is an essential part of the invention and comprises:
- tabs (16) that arise preferably from the coating (13); and
- nails (15) in the end of the tabs (16).

The tabs (16), as can be seen in figure 1, allow the forward and backward movement of the fibre (14) in the inner wall (19) of the blood vessel (17), as the end (20) of the body (13) has been designed so that the tabs (16) of length L1 and height h1, have nail-shaped end parts (15) of length L2 and height h1, as can be seen in detail in figure 2.

The function of the end part (20) of the body (13) is to maintain sufficient distance "d1", by taking into consideration the total diameter "d2" of the blood vessel (17), and in this manner to enable the movement of the catheter (11) with the optic fibre cable (14) inside, without damaging the vessel or getting stuck when the vessel (17) has curves of different radius, when it could damage or even perforate (17) the fibre (14) loses the central position, with this aim, as can be seen in the views (c) and (d) of figure 2, all the tabs (16) display in their cross-section a curvature of radius R1.

As shown in figures 1 and 2a, the inner portions (16a) of the tabs (16) have a straight longitudinal section, in the same manner as the end portions or nails (15) of the tabs, forming an angle between these inner portions and the end portions or nails (15).

After carrying out many tests with the object described in the invention, the conclusion has been that the best dimensions for the tip are:
- for length L1 with regard to the longitudinal axis: between 1 and 30 mm. and/or
- for length L2 with regard to the longitudinal axis: between 1 and 30 mm. and/or
- For the radius of curvature R1 is above 0 and preferably between 0.5 and 10 mm.

The purpose of the end part (20) is to self-centre the optical fibre output in order to avoid touching the vessel wall (17) at any curvature or in the irregularities diameter and lo perforate it. Additionally, it allows the optical fibre assembly to move forward and backwards inside the vessel (17).

Having sufficiently described this invention using the attached drawings, it is easy to understand that any changes judged to be suitable may be made, whenever these changes do not alter of the essence of the invention summarised in the following claims.

## Claims

1. **A BIDIRECTIONAL SELF-CENTRING TIP FOR FIBRE OPTICS DESIGNED FOR ENDOVASCULAR TREATMENTS"** which comprise a cover surrounded by a jacket and covers concentric to the optical fibre, this jacket being a tubular duct the end part of which has straight fingers that for an open cone shape, the straight ends of which maintain a certain distance between the optical fibre and the inner wall of the vessel, the optical fibre and its core receiving laser energy by suitable means that is projected onto the inner face of the blood vessel, **characterised in that** the body of the self-centring and bidirectional tip of the fibre optic cable, formed by a tubular duct the end of which has tabs that extend outward in a conical direction, and these tabs in their respective end parts comprise respective nails that are sloping with regard to the tabs towards the longitudinal axis of the vessel.

2. **A BIDIRECTIONAL SELF-CENTRING TIP FOR FIBRE OPTICS DESIGNED FOR ENDOVASCULAR TREATMENTS"** according to the claim 1, **characterised in that** tabs have a length with regard to the longitudinal axis L1 and a height with regard to the longitudinal axis h1, whereas nails have a length with regard to the longitudinal axis L2 and a height with regard to the longitudinal axis h1.

3. **A BIDIRECTIONAL SELF-CENTRING TIP FOR FIBRE OPTICS DESIGNED FOR ENDOVASCULAR TREATMENTS"** according to the preceding claim, **characterised in that** the length L1 is between 1 and 30 mm.

4. A **BIDIRECTIONAL SELF-CENTRING TIP FOR FIBRE OPTICS DESIGNED FOR ENDOVASCULAR TREATMENTS"** according to the claim 2, **characterised in that** the length L2 is between 2 and 30 mm.

5. **A BIDIRECTIONAL SELF-CENTRING TIP FOR FIBRE OPTICS DESIGNED FOR ENDOVASCULAR TREATMENTS"** according to any of claims 1 to 4, **characterised in that** the inner portions of the tabs have a straight longitudinal section.

6. **A BIDIRECTIONAL SELF-CENTRING TIP FOR FIBRE OPTICS DESIGNED FOR ENDOVASCULAR TREATMENTS"** according to any of claims 1 to 4, **characterised in that** the end portions (nails) of the tabs have a straight longitudinal section.

7. **A BIDIRECTIONAL SELF-CENTRING TIP FOR FIBRE OPTICS DESIGNED FOR ENDOVASCULAR TREATMENTS**" according to any of the preceding claims, **characterised in that** the tabs have a cross-section with side bases with concave radius of curvature R1 greater than 0.

8. **A BIDIRECTIONAL SELF-CENTRING TIP FOR FIBRE OPTICS DESIGNED FOR ENDOVASCULAR TREATMENTS"** according to the claim 7, **characterised in that** R1 is between 0.5 and 10 mm.

9. **A BIDIRECTIONAL SELF-CENTRING TIP FOR FIBRE OPTICS DESIGNED FOR ENDOVASCULAR TREATMENTS"** according to the claim 1, **characterised in that** the end part of the optical fibre has a uniform conical shape.
